# EUROPÄISCHE PATENTSCHRIFT

(11) **EP 2 587 993 B1**
(45) Veröffentlichungstag und Bekanntmachung des Hinweises auf die Patenterteilung: **11.10.2017**
(21) Anmeldenummer: 11728854.8
(22) Anmeldetag: 01.07.2011
(51) Int. Cl.: A61B 8/00, G01R 33/48, A61B 5/055, A61B 8/02, A61B 8/08, A61B 5/03, G01R 33/567

(54) **ULTRASCHALLSENSOR FÜR EINEN KARDIOTOKOGRAPHEN**
ULTRASONIC SENSOR FOR A CARDIOTOCOGRAPH
CAPTEUR À ULTRASONS POUR CARDIOTOCOGRAPHE

(30) Priorität: 02.07.2010 DE 102010025857
(43) Veröffentlichungstag der Anmeldung: 08.05.2013
(73) Patentinhaber: Wedegärtner, Ulrike, 22453 Hamburg (DE)
(72) Erfinder: WEDEGÄRTNER, Ulrike, 22453 Hamburg (DE); VALETT, Klaus, 25373 Ellerhoop (DE); YAMAMURA, Jin, 20255 Hamburg (DE)
(74) Vertreter: Schwarz, Markku
(86) Internationale Anmeldenummer: PCT/EP2011/061131
(87) Internationale Veröffentlichungsnummer: WO 2012/001150

(56) Entgegenhaltungen:
- WO-A1-93/08534
- WO-A1-95/02361
- WO-A1-2004/026135
- WO-A2-2007/059474
- WO-A2-2008/097487
- US-A1- 2005 272 995

## Beschreibung

### Gebiet der Erfindung

Die Erfindung betrifft einen Ultraschallsensor für Kardiotokographie (CTG). Insbesondere betrifft die Erfindung einen CTG-Ultraschallsensor, welcher in einem Magnetresonanztomographen (MRT) verwendbar ist. Ferner betrifft die Erfindung einen Kardiotokographen, d.h. ein CTG-Gerät, mit einem solchen Ultraschallsensor sowie ein System aus einem MRT und einem CTG-Gerät mit einem solchen Ultraschallsensor.

### Hintergrund der Erfindung

Da das fetale Herz innerhalb des Uterus liegt, ist dort nicht die Möglichkeit gegeben für eine direkte Erfassung der fetalen Herzfrequenz und damit ist die üblicherweise benötigte Elektrokardiogramm-Steuerung bei Luftanhalten des Patienten während der Messung nicht möglich. Daher ist die Visualisierung von Anomalien des Herzens und großer Gefäße durch MRI (Magnetresonanzbildgebung) nicht erreichbar.

Für die Untersuchung eines fetalen Herzens mittels MRT beschreiben beispielsweise Manganaro et al., Prenat. Diagn. 2008, 28, 148-156 und Fogel et al., Fetal Diagn. Ther. 2005, 20, 475-480 True fast imaging with steady-state precision (True FISP) und real-time cine-MRsequences zu verwenden, wobei die Durchführung hierbei allerdings ohne eine Steuerung (Triggerung) erfolgt. Nijm et al., J. Magn. Reson. Imaging 2008, 28, 767-772 nutzen selfgating (SG)-Algorithmen zur Synchronisation. Hierbei ist unter anderem das niedrige signalto-noise-Verhältnis limitierend. Yamamura et al., Eur. Radiol. 2009, 19, 2383-90 nutzen ein invasives Triggersystem (Pulse wave triggering). Alle diese Methoden weisen starke Einschränkungen auf, die entweder ihre praktische Anwendung am Menschen unmöglich machen oder es werden keine Bilder ausreichender Qualität geliefert, die die Auswertung von anatomischen Strukturen und funktionalen Informationen erlauben. Michel et al., American Journal of Roentgenologie 2003, 180, 1159-1164, kommen zwar zu dem Schluss, dass die fetale CTG während der Magnetresonanztomographie mit modifiziertem Standard-Equipment machbar ist. Allerdings wird auch festgestellt, dass aus technischen Gründen das CTG-Monitoring während des Aufenthalts des Patienten im Magneten nicht möglich ist (stattdessen wurden Messungen direkt nach dem Verlassen des Magneten getätigt). Die Studie von Michel et al. liefert entsprechend keine MRT-Bilder des fetalen Herzens.

Die WO 95/02361 beschreibt ein MRT-System, bei welchem ein Ultraschallsensor verwendet werden kann, um Blutflussgeschwindigkeitsinformationen bereitzustellen und darüber ein Trigger-Signal für die MRT-Bildgebung zu gewinnen. Hierfür wird ein aus nichtmagnetischen Materialien hergestellter Ultraschallsensor verwendet. Der Ultraschallsensor kommt außerhalb des Bildgebungsbereichs des MRT, insbesondere im Bereich der KarotisArterie zum Einsatz.

Die Verwendung von ultraschallbasierten Sensoren zur Triggerung der Bildgebung eines MRT-Systems wird auch in der US 2005/0272995 A1 beschrieben. In diesem Fall wird ein Ultraschallschwinger beispielsweise auf dem Brustkorb des Patienten angeordnet, um Herzbewegungen oder den Blutfluss zu erfassen.

Die WO 2004/026135 A1 beschreibt Verdrahtungsanordnungen für Sensorkatheter, bei welchen verdrillte Leitungspaare zum Einsatz kommen, um elektromagnetische Interferenz unter verschiedenen Leitungen zu vermindern.

Die WO 2007/059474 A2 bezieht sich auf die Reduzierung von Störungen in einem Bildgebungskathetersystem, bei welchem Ferrit-Kerne zur Vermeidung von Signalverzerrungen eingesetzt werden.

Die WO 2008/097487 A2 beschreibt ein Ultraschallbildgebungssystem, bei welchem eine Basiseinheit und ein Sensor vorgesehen sind, welche drahtlos kommunizieren können. Insbesondere ist vorgesehen, dass über Ultraschallschwinger aufgenommene Signale an die Basiseinheit übertragen werden, wo eine weitere Auswertung zur Bildgebung erfolgt.

Das Problem der störenden Wechselwirkung zwischen dem CTG-Gerät und dem MRT ist ein seit Jahren ungelöstes Problem.

### Zusammenfassung der Erfindung

Es ist eine Aufgabe der Erfindung, einen Ultraschallsensor für ein CTG-Gerät bereitzustellen, welcher geeignet ist, ein Steuersignal für eine Untersuchung eines Patienten in einem MRT bereitzustellen. Diese und weitere Aufgaben werden durch die jeweiligen Gegenstände der unabhängigen Ansprüche gelöst. Weitere bevorzugte Ausführungsformen sind in den abhängigen Ansprüchen beschrieben.

Gemäß einem Ausführungsbeispiel der Erfindung weist ein Ultraschallsensor für ein Cardiotokographiegerät (CTG-Gerät) somit zumindest einen Ultraschallschwinger, zumindest einen Widerstand, der mit dem Ultraschallschwinger verbunden ist, und ein Gehäuse auf. In dem Gehäuse sind der Ultraschallschwinger und der Widerstand untergebracht. Der Ultraschallsensor ist aus Materialien hergestellt, die nicht ferromagnetisch sind, so dass sich der Ultraschallsensor neutral gegenüber einem äußeren Magnetfeld verhält. Speziell bedeutet dies, dass der Widerstand aus einem nicht-ferromagnetischen Material besteht und dass der Widerstand mittels eines nicht-ferromagnetischen Drahtes mit dem Ultraschallschwinger verbunden ist.

Der Ultraschallschwinger ist über verdrillte Kabel mit dem Widerstand verbunden. Genauer gesagt kann ein erster Pol des Ultraschallschwingers mit einem Signal-Leiter einer CTG-Elektronik des CTG-Geräts verbunden sein und ein zweiter Pol des Ultraschallschwingers ist über den Widerstand mit einer Masse der CTG-Elektronik verbunden, wobei die beiden Kabel miteinander verdrillt sind.

Selbst wenn der Ultraschallsensor Signale aufnimmt und selbst innerhalb des Magnetfelds eines MRT angeordnet ist, wird das Magnetfeld des MRT nahezu nicht gestört oder beeinflusst, so dass eine Bildgebung durch das MRT nahezu ohne Artefakte und in der gewünschten Auflösung möglich ist. Beispielsweise kann der Ultraschallsensor die Bildgebung in seiner unmittelbaren Nähe beeinflussen, ohne dass diese Beeinflussung den Bereich von Interesse (zum Beispiel das fetale Herz) betrifft. Weiterhin werden Störungen des Betriebs des Ultraschallsensors durch das Magnetfeld des MRT und die von dem MRT ausgestrahlten Hochfrequenzimpulse vermieden. Somit kann der Ultraschallsensor auch während laufender Messungen im Bildgebungsbereich des MRT betrieben werden. Es ist so insbesondere möglich dopplersonografische Signale im MRT während laufender MRT-Messungen aufzunehmen.

Der Ultraschallsensor ist auf der einen Seite neutral gegenüber dem äußeren Magnetfeld, d. h. er erzeugt keine Störungen im Magnetfeld. Auf der anderen Seite ist der Ultraschallsensor durch die Verbindung über verdrillte Kabel aber auch gegen Störungen des weitaus stärkeren äußeren Magnetfeldes abgeschirmt. Diese gute Abschirmung erlaubt eine vollständige Funktionsfähigkeit des Ultraschallsensors sowohl im statischen Magnetfeld als auch während der laufenden MRT-Messungen mit Einstrahlung von Hochfrequenzimpulsen.

'Neutral' im Sinne der Erfindung umfasst, dass ein mögliches störendes Feld lediglich auftritt in einem Abstand von bis zu 30 mm, gemessen senkrecht zur Oberfläche von beispielsweise dem Gehäuse des Ultraschallsensors. Bevorzugt tritt ein störendes Feld nur bis zu einem Abstand von 20 mm auf. Idealerweise wird ein äußeres Magnetfeld nur im Bereich eines Abstandes von 0 bis 12 mm gestört oder beeinflusst.

Gemäß einem Ausführungsbeispiel der Erfindung weist der Ultraschallsensor keine Platine auf, über welche der zumindest eine Ultraschallschwinger und der zumindest eine Widerstand miteinander verbunden sind. Auf diese Weise kann eine weitere störende Struktur vermieden werden.

Gemäß einem Ausführungsbeispiel der Erfindung weist der Ultraschallsensor sieben Ultraschallschwinger und sieben Widerstände auf, welche beispielsweise über eine freie Bedrahtung, vorzugsweise mit paarweise verdrillten Kabeln, miteinander verbunden sind.

Die Widerstände wie auch die Bedrahtung können aus einem oder mehreren nicht-ferromagnetischen Materialien bestehen. Insbesondere eisen- und nickelhaltige Materialien können auf diese Weise vermieden werden.

Gemäß einem weiteren Ausführungsbeispiel der Erfindung können der Ultraschallschwinger, der Widerstand sowie die verdrillte Bedrahtung auf einer nicht-ferromagnetischen Platine angeordnet sein, die entsprechend frei von Eisen oder Nickel etc. sein sollte. Über eine derartige Platine kann eine automatisierbare Herstellung erleichtert werden. Außerdem kann eine zuverlässige Anordnung von mehreren Elementen gewährleistet werden.

Als Abschirmung kann das Gehäuse des Ultraschallsensors von außen komplett metallisiert sein, beispielsweise mit Leitsilber behandelt sein.

Erfindungsgemäß ist eine kabellose Übertragung vorgesehen. Insbesondere ist die CTG-Elektronik in dem Gehäuse des Ultraschallsensors integriert, und ein einen Herzrhythmus repräsentierendes Ausgangssignal der CTG-Elektronik kann mittels einer Sendeeinheit kabellos an das MRT übertragen werden, von welchem es beispielsweise als Triggersignal für die Herzbildgebung verwendet werden kann. Vorzugsweise ist auch die CTG-Elektronik durch nicht-ferromagnetische Materialien realisiert und verwendet eine Bedrahtung mit paarweise verdrillten Kabeln, um Störungen durch dynamische elektrische und magnetische Felder im Betrieb des MRT zu vermeiden. Weiterhin kann die CTG-Elektronik in diesem Fall auch Signalfilter zur Unterdrückung von Frequenzen der von dem MRT eingestrahlten Hochfrequenzsignale umfassen.

Jede der oben beschriebenen einzelnen Maßnahmen der Abschirmung sorgt für eine verbesserte Kompatibilität des Ultraschallsensors mit dem MRT.

Gemäß einem weiteren Ausführungsbeispiel der Erfindung kann ein Ultraschallsensor gemäß einem oder mehreren der genannten Ausführungsbeispiele zusammen mit einem MRT verwendet werden, wobei der Ultraschallsensor innerhalb des Magnetfeldes des MRT angeordnet ist und wobei die Bildgebung des MRT durch das Ausgangssignal der CTG-Elektronik gesteuert (getriggert) wird.

Gemäß einem weiteren Ausführungsbeispiel der Erfindung wird ein System zur Bildgebung eines Herzens, insbesondere eines fetalen Herzens, bereitgestellt, welches den Ultraschallsensor, ein CTG-Gerät mit dem ultraschallsensor, sowie ein MRT aufweist, wobei die CTG-Elektronik angepasst ist, auf der Basis einer Schwingungserfassung des Ultraschallschwingers das Ausgangssignal zur Steuerung der Bildgebung des MRT bereitzustellen.

Hierfür kann die CTG-Elektronik mehrfach spezielle Impulsketten (Bursts) an den Sensor senden, die dieser als Ultraschall abstrahlt. Dann kann jeweils das Echo der Bursts empfangen und von der CTG-Elektronik interpretiert werden, wobei die Interpretation bei dieser Anwendung auf Laufzeitunterschieden beruhen kann (Doppler-Effekt), aus denen dann die Herzfrequenz berechnet werden kann.

Die oben beschriebenen Aspekte und weitere Aspekte, Merkmale und Vorteile der Erfindung können ebenfalls aus den Beispielen der Ausführungsformen entnommen werden, welche im Folgenden unter Bezugnahme der anhängenden Zeichnungen beschrieben werden.

### Kurze Beschreibung der Zeichnungen

Fig. 1 ist eine schematische Darstellung eines Ultraschallsensors.
Fig. 2 ist eine vergrößerte Darstellung des Details X aus Fig. 1.
Fig. 3 ist eine schematische Darstellung eines Systems mit CTG und MRT.

### Detaillierte Beschreibung der Ausführungsformen

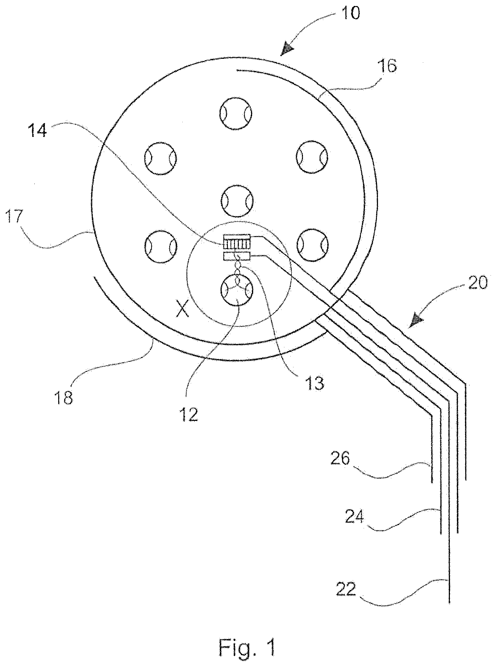

Fig. 1 zeigt eine schematische Darstellung eines Ultraschallsensors 10 mit einem CTG-Kabel 20. Der Ultraschallsensor liefert ein Triggersignal zur verbesserten Bildgebung eines MRT.

Der Ultraschallsensor 10 weist ein Gehäuse 17 auf, in welchem Ultraschallschwinger 12 und Widerstände 14 angeordnet sind. In Fig. 1 sind exemplarisch ein Ultraschallschwinger 12 und ein Widerstand 14 über eine verdrillte Bedrahtung 13 miteinander verbunden (Detail X). Der Übersicht wegen ist auf eine Darstellung der verdrillten Bedrahtungen zwischen den anderen Ultraschallschwingern und den korrespondierenden Widerständen verzichtet worden.

Ferner ist innerhalb des Gehäuses 17 eine Abschirmfolie 16 vorgesehen. Die Abschirmfolie 16 ist in Fig. 1 lediglich schematisch angedeutet. Es wird angemerkt, dass die Abschirmfolie derart ausgebildet sein kann, dass sie alle Ultraschallschwinger 12 und auch die Widerstände 14 sowie die Bedrahtungen 13 innerhalb des Gehäuses 17 abdeckt.

Das Äußere des Gehäuses 17 ist komplett metallisiert, beispielsweise mit Leitsilber 18 behandelt. Auch hier ist das Leitsilber 18 nur teilweise und schematisch in Fig. 1 angedeutet. Es wird angemerkt, dass das Leitsilber 18 die komplette äußere Oberfläche des Gehäuses 17 abdecken kann.

Von dem Gehäuse 17 des Ultraschallsensors 10 führt ein CTG-Kabel 20 weg. Das CTG-Kabel 20 besteht aus einem Kern 22, einer inneren Abschirmung 24 und einer äußeren Abschirmung 26. Der innere Kern 22 ist innerhalb des Ultraschallsensors mit der Bedrahtung 13 der Widerstände 14 und der Ultraschallschwinger 12 verbunden, so dass ein Signal von den Ultraschallschwingern aus dem Ultraschallsensor zu einer CTG- Elektronik abgeleitet werden kann. Der Kern des CTG-Kabels 20 kann zweipolig ausgeführt sein.

Die innere Abschirmung 24 führt auf der Seite des Ultraschallsensors bis in das Gehäuse 17 hinein und ist in dem Gehäuse mit der Abschirmfolie 16 verbunden. Die innere Abschirmung 24 ist über die gesamte Länge des Kabels 20 vorgesehen und ist an ihrem anderen Ende mit der Masse der CTG-Elektronik verbunden. Auf diese Weise ist die Abschirmfolie in dem Gehäuse 17 des Ultraschallsensors mit der Masse der CTG-Elektronik verbunden.

Die äußere Abschirmung 26 des CTG-Kabels 20 ist nicht über die gesamte Länge des CTG-Kabels ausgebildet. Beispielsweise können 1,5 m des CTG-Kabels ausgehend vom Ultraschallsensor 10 mit der äußeren Abschirmung 26 ausgebildet sein. Diese zusätzliche äußere Abschirmung ist mit der Masse des MRT verbunden und an dem Ultraschallsensor mit dem Leitsilber 18 außen an dem Gehäuse 17 verbunden.

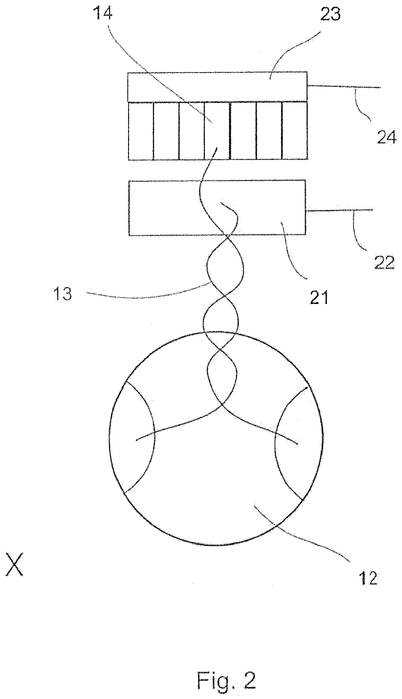

Fig. 2 zeigt das Detail X aus Fig. 1 als vergrößerte Darstellung. Der Ultraschallschwinger 12 ist über eine verdrillte Bedrahtung 13 einerseits mit dem Kern 22 des CTG-Kabels als Signal-Leiter verbunden und andererseits über einen Widerstand 14 mit der inneren Abschirmung 24 des CTG-Kabels. Der Widerstand 14 bildet einen Teil eines Blocks, der aus sieben SMD-Widerständen besteht, wobei diese sieben Widerstände einen gemeinsamen Widerstand-Kontakt 23 zu der Abschirmung 24 haben und einen jeweiligen freien Kontakt.

Einer der zwei Drähte 13, die von dem Ultraschallschwinger 12 kommen, ist mit einem freien Kontakt eines Widerstandes 14 verbunden und der andere der zwei Drähte ist mit dem Kern 22 verbunden, wobei die Signal-Kontaktstelle 21 so ausgebildet ist, dass alle Ultraschallschwinger mit dieser Kontaktstelle verbunden sein können.

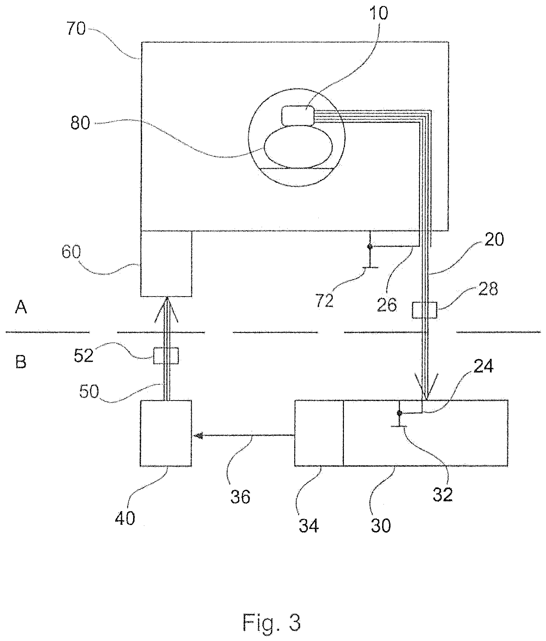

Fig. 3 zeigt eine schematische Darstellung eines Systems zur Bildgebung eines Herzens, insbesondere eines fetalen Herzens. Der Ultraschallsensor 10 ist über das CTG-Kabel 20 mit einer CTG-Elektronik 30 verbunden. Auch hier in Fig. 3 ist angedeutet, dass die innere Abschirmung 24 des Kabels 20 mit der Masse 32 der CTG-Elektronik 30 verbunden ist. Ferner ist dargestellt, dass die äußere Abschirmung 26 mit der Masse 72 des MRT 70 verbunden ist. Zusätzlich ist dargestellt, dass um das CTG-Kabel 20 herum ein Ferritkern 28 vorgesehen ist.

Bei der CTG-Elektronik 30 kann, wie bei einem handelsüblichen CTG-Gerät, im Betrieb ein Leuchtfeld in der Frontplatte im Herzrhythmus blinken. Um dieses blinkende Leuchtfeld zu nutzen, kann ein Opto-Koppler 34 eingesetzt werden, der aus dem Signal des Leuchtfeldes ein elektronisches Signal erzeugt, welches einen Herzrhythmus repräsentiert.

Alternativ kann auch ein CTG-Gerät verwendet werden, welches ein elektrisches Ausgangssignal liefert, das einen Herzrhythmus repräsentiert. Die CTG-Elektronik 30 ist mit dem Ultraschallsensor 10 in demselben Gehäuse integriert, z.B. in Form eines Handgerätes. Das Ausgangssignal der CTG-Elektronik 30 wird kabellos an das MRT 70 übertragen, z.B. über Funksignale, Infrarotsignale oder akustische Signale.

Bei dem in Fig. 3 dargestellten Beispiel wird das elektronische Signal des Opto-Koppters 34 überein Kabel 36 zu einer Elektronik 40 weitergeleitet, welche das Signal in ein EKG-ähnliches, sehr niederohmiges Signal wandelt. Über ein Koaxialkabel 50, an welchem ein weiterer Ferritkern 52 vorgesehen sein kann, wird dieses gewandelte Signal an eine weitere Elektronik 60 weitergeleitet, welche eine Signalpegel-Anpassung vornimmt.

Das resultierende, aufbereitete Signal kann nun vom MRT 70 als Steuersignal (Triggersignal) für die Herzbildgebung verwendet werden. Auf diese Weise können MRT-Darstellungen des Herzens eines Patienten 80 durchgeführt werden, die immer zum gleichen Zeitpunkt in einem Herzzyklus aufgenommen sind, so dass anatomische Strukturen des Herzens mit sehr hoher Auflösung dargestellt werden können. Dies ist mittels des erfindungsgemässen Systems insbesondere auch für ein Herz eines ungeborenen Kindes im Mutterleib möglich. Es wird angemerkt, dass auch der Ablauf der Herzbewegungen darstellbar ist, wobei hierfür das Steuersignal einen fortschreitenden Zeitpunkt relativ zum Herzzyklus für die Bildgebung vorgibt.

Es versteht sich, dass bei den dargestellten Beispielen verschiedene Modifikationen möglich sind. Der Ultraschallsensor und die CTG-Elektronik sind nicht als separate Komponenten vorgesehen, sondern sind in demselben Gehäuse, z.B. dem Gehäuse 17 des in Fig. 1 dargestellten Ultraschallsensors 10. integriert. Beispielsweise können der Ultraschallsensor und die CTG-Elektronik in einem Handgerät bzw. Kompaktgerät zusammengefasst sein. Die erforderliche Hardware und Software zur Signalgenerierung und -aufbereitung könnte dann in diesem Kompaktgerät implementiert sein, welches wie der Ultraschallsensor MRT-kompatibel ausgestaltet ist. Das MRT-kompatible Kompaktgerät kann beispielsweise auf dem Patienten über dem zu untersuchenden Objekt platziert werden, und die gemessenen Signale können kabellos an das MRT weitergeleitet werden. Auf ein spezielles CTG-Kabel, z.B. mit Masseableitung am MRT-Gerät, könnte dann verzichtet werden.

Weiterhin versteht es sich, dass die hierin beschriebenen Konzepte in einer Vielzahl von Einsatzbereichen Vorteile bieten. Beispiele von solchen Einsatzbereichen sind:
- Erzeugung eines Triggersignals, welches die Herzfrequenz von Erwachsenen, Kindern und intrauterin von Feten repräsentiert. Dieses Triggersignal kann zur Herz- und Gefäßbildgebung im MRT verwendet werden. Die dadurch ermöglichte getriggerte kardiovaskuläre fetale MRT-Bildgebung liefert wertvolle Informationen für das weitere therapeutische Vorgehen bei fetalen Fehlbildungen. Das MRT erlaubt eine genaue anatomische Darstellung des Herzens (inklusive Foramen ovale) und zusätzlich funktionelle Aussagen wie z.B. die Ejektionsfraktion. So kann bereits intrauterin das Ausmaß einer kardialen Fehlbildung für die Planung nachfolgender chirurgischer Eingriffe bestimmt werden. Abgesehen vom Einsatz in der Pränataldiagnostik kann das System auch die EKG-Steuerung bei der Untersuchung von Erwachsenen ersetzen. Es kann dann für die Untersuchung von Erwachsenen vorteilhaft genutzt werden, wenn die üblichen EKG-Elektroden grundsätzlich Mehraufwand (Rasieren der Brust) und Probleme (Abfallen der selbstklebenden EKG-Elektroden) verursachen und in manchen Fällen gar nicht eingesetzt werden können (z.B. bei Patienten mit Pleuraergüssen, Perikardergüssen, Adipositas).
- Monitoring der Herzfrequenz von Feten, Kindern, Erwachsenen und damit einer Vitalfunktion während der MRT-Messung: Neben der cardialen Triggerung eignet sich das hierin beschriebene MRT kompatible CTG-Gerät auch zum kontinuierlichen Monitoring von Feten während der MRT-Untersuchung. Dies ist von klinischer Bedeutung, da häufig Risikoschwangerschaften im MRT untersucht werden. Natürlich kann ein solches Monitoring auch bei Kindern oder Erwachsenen erfolgen.

### Bezugszeichenliste

- 10: Ultraschallsensor
- 12: Ultraschallschwinger
- 13: Bedrahtung
- 14: Widerstand
- 16: Abschirmfolie
- 17: Gehäuse
- 18: Leitsilber
- 20: CTG-Kabel
- 21: Signal-Kontaktstelle
- 22: Kabel-Kern
- 23: Widerstand-Kontakt
- 24: Innere Abschirmung
- 26: Äußere Abschirmung
- 28: Ferritkern
- 30: CTG-Elektronik
- 32: CTG-Masse
- 34: Opto-Koppler
- 36: Kabel
- 40: Signalwandler
- 50: Koaxialkabel
- 52: Ferritkern
- 60: Signalpegelanpassung
- 70: MRT
- 72: MRT-Masse
- 80: Patient

## Patentansprüche

1. Ultraschallsensor (10) für ein Kardiotokographiegerät, aufweisend:
einen Ultraschallschwinger (12),
einen Widerstand (14), und
ein Gehäuse (17), in welchem der Ultraschallschwinger und der Widerstand untergebracht sind,
wobei der Ultraschallsensor (10) aus einem nicht-ferromagnetischen Material hergestellt ist, so dass er sich neutral gegenüber einem äußeren Magnetfeld verhält, wobei der Widerstand mit dem Ultraschallschwinger über verdrillte Kabel verbunden ist,
eine Kardiotokographie-Elektronik (30) in dem Gehäuse (17) untergebracht ist, wobei die Kardiotokographie-Elektronik ein Ausgangssignal liefert, welches einen Herzrhythmus repräsentiert, und
der Ultraschallsensor (10) eine Sendeeinheit zur kabellosen Übertragung des Ausgangssignals an einen Magnetresonanztomographen (70) aufweist.

2. Verwendung eines Ultraschallsensors (10) gemäß Anspruch 1 mit einem Magnetresonanztomographen (70), wobei der Ultraschallsensor (10) innerhalb des Magnetfeldes des Magnetresonanztomographen (70) angeordnet ist, und
wobei die Bildgebung des Magnetresonanztomographen durch das Ausgangssignal der Kardiotokographie-Elektronik (30) gesteuert wird.

3. Verwendung gemäß Anspruch 2,
wobei das Ausgangssignal einen Herzrhythmus eines Erwachsenen, eines Kindes, oder eines Feten im Mutterleib repräsentiert.

4. Verwendung gemäß Anspruch 2,
wobei der Magnetresonanztomograph (70) zur Herz- oder Gefäßbildgebung eingesetzt wird.

5. Kardiotokographiegerät mit
einem Ultraschallsensor (10) gemäß Anspruch 1.

6. Kardiotokographiegerät gemäß Anspruch 5,
wobei der Ultraschallsensor (10) in einem Magnetfeld des Magnetresonanztomographen (70) anordenbar ist, wobei die Anwesenheit des Ultraschallsensors (10) die Bildgebung des Magnetresonanztomographen (70) nicht störend beeinflusst, und
wobei die Bildgebung des Magnetresonanztomographen (70) durch das Ausgangssignal steuerbar ist, welches von der Kardiotokographie-Elektronik (30) erzeugt wird.

7. System zur Bildgebung eines Herzens, aufweisend
einen Ultraschallsensor (10) gemäß Anspruch 1, und
einen Magnetresonanztomographen (70),
wobei die Kardiotokographie-Elektronik (30) angepasst ist, auf der Basis einer Schwingungserfassung des Ultraschallschwingers des Ultraschallsensors (10) das Ausgangssignal zur Steuerung der Bildgebung des Magnetresonanztomographen (70) bereitzustellen.

## Claims

1. An ultrasonic sensor (10) for a cardiotocography device, comprising:
an ultrasonic transducer (12),
a resistor (14), and
a housing (17) accommodating the ultrasonic transducer and the resistor,
wherein the ultrasonic sensor (10) is formed of a non-ferromagnetic material, so that it acts neutrally with respect to an external magnetic field,
wherein
the resistor is connected to the ultrasonic transducer by twisted wires,
a cardiotocography electronic system (30) is accommodated in the housing (17), the cardiotocography electronic system providing an output signal which represents a heart rhythm, and
the ultrasonic sensor (10) comprises a transmit unit for wireless transmission of the output signal to a magnetic resonance tomograph.

2. Use of an ultrasonic sensor (10) according to claim 1 with a magnetic resonance tomograph (70), wherein the ultrasonic sensor (10) is arranged within the magnetic field of the magnetic resonance tomograph (70), and
wherein the imaging of the magnetic resonance tomograph is controlled by the output signal of the cardiotocography electronic system (30).

3. Use according to claim 2,
wherein the output signal represents a heart rhythm of an adult, a child or a fetus in the mother's womb.

4. Use according to claim 2,
wherein the magnetic resonance tomograph (70) is applied for heart or vascular imaging.

5. A cardiotocography device comprising
an ultrasonic sensor (10) according to claim 1.

6. Cardiotocography device according to claim 5,
wherein the ultrasonic sensor (10) is arrangeable in a magnetic field of the magnetic resonance tomograph (70),
wherein the presence of the ultrasonic sensor (10) does not adversely affect the imaging of the magnetic resonance tomograph (70), and
wherein the imaging of the magnetic resonance tomograph (70) is controllable by the output signal, which is generated by the cardiotocography electronic system (30).

7. System for imaging of a heart, comprising
an ultrasonic sensor (10) according to claim 1, and
a magnetic resonance tomograph (70),
wherein the cardiotocography electronic system (30) is configured to provide the output signal for controlling the imaging of the magnetic resonance tomograph (70) on the basis of oscillation detection by the ultrasonic transducer of the ultrasonic sensor (10).

## Revendications

1. Capteur à ultrasons (10) pour dispositif de cardiotocographie, comportant:
un oscillateur à ultrasons (12),
une résistance (14), et
un boîtier (17) logeant l'oscillateur à ultrasons et la résistance,
le capteur à ultrasons (10) étant fabriqué d'un matériau non-ferromagnétique de sorte à avoir un comportement neutre vis-à-vis d'un champ magnétique extérieur,
la résistance étant reliée à l'oscillateur à ultrasons par l'intermédiaire de câbles torsadés,
une électronique de cardiotocographie (30) étant logée dans le boîtier (17), l'électronique de cardiotocographie délivrant un signal de sortie représentant un rythme cardiaque, et
le capteur à ultrasons (10) comportant une unité d'émission pour la transmission sans fil du signal de sortie vers un tomographe à résonance magnétique (70).

2. Utilisation d'un capteur à ultrasons (10) selon la revendication 1 avec un tomographe à résonance magnétique (70), le capteur à ultrasons (10) étant disposé au sein du champ magnétique du tomographe à résonance magnétique (70), et
l'imagerie du tomographe à résonance magnétique étant pilotée par le signal de sortie de l'électronique de cardiotocographie (30).

3. Utilisation selon la revendication 2, dans laquelle le signal de sortie représente un rythme cardiaque d'un adulte, d'un enfant ou d'un foetus in utero.

4. Utilisation selon la revendication 2, dans laquelle le tomographe à résonance magnétique (70) est mis en oeuvre pour une imagerie du coeur ou des vaisseaux.

5. Dispositif de cardiotocographie avec un capteur à ultrasons (10) selon la revendication 1.

6. Dispositif de cardiotocographie selon la revendication 5, le capteur à ultrasons (10) pouvant être disposé dans un champ magnétique du tomographe à résonance magnétique (70), la présence du capteur à ultrasons (10) ne perturbant pas l'imagerie du tomographe à résonance magnétique (70), et
l'imagerie du tomographe à résonance magnétique (70) étant apte à être pilotée par le signal de sortie engendré par l'électronique de cardiotocographie (30).

7. Système d'imagerie d'un coeur, comportant
un capteur à ultrasons (10) selon la revendication 1, et un tomographe à résonance magnétique (70),
l'électronique de cardiotocographie (30) étant adaptée à fournir le signal de sortie pour piloter l'imagerie du tomographe à résonance magnétique (70) à base d'une détection d'oscillation de l'oscillateur à ultrasons du capteur à ultrasons (10).
